# EUROPEAN PATENT APPLICATION

(11) **EP 4 501 905 A1**
(43) Date of publication of application: **05.02.2025**
(21) Application number: 23781047.8
(22) Date of filing: 31.03.2023
(51) Int. Cl.: C07C 69/74, C10M 105/36, C10N 30/02, C10N 30/06, C10N 40/00

(54) **COMPOUND, LUBRICATING OIL BASE OIL, AND LUBRICATING OIL COMPOSITION**

(30) Priority: 31.03.2022 JP 2022061445
(71) Applicant: Idemitsu Kosan Co.,Ltd., Tokyo 100-8321 (JP)
(72) Inventor: YOSHIDA Yukio, Tokyo 100-8321 (JP); NAKANISHI Yusuke, Tokyo 100-8321 (JP)
(74) Representative: Hoffmann Eitle
(86) International application number: PCT/JP2023/013511
(87) International publication number: WO 2023/191039

(57) **Abstract**

Provided is a compound represented by General Formula (A-1) below. Also provided is lubricant base oil including the compound represented by General Formula (A-1) below. Further provided is a lubricating oil composition including the lubricant base oil. [In General Formula (A-1) above, R₁₁, R₁₂, and R₁₃ are each independently a chained alkyl group having 4 to 30 carbon atoms.] Consequently, a compound, lubricant base oil, and a lubricating oil composition which have low viscosity and a high viscosity index and are excellent in wear resistance and reduction in friction coefficient can be provided.

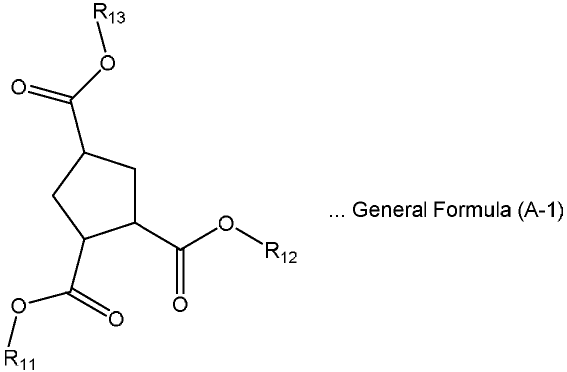

## Description

### Technical Field

The present invention relates to a compound, lubricant base oil, and a lubricating oil composition.

### Background Art

Unlike the earth's environment, cosmic space has no atmosphere, thus has no atmospheric pressure, and is an extremely high-vacuum environment. In such a high-vacuum environment, a liquid with a high vapor pressure evaporates during use and thus is difficult to use in cosmic space for a long period. In addition, surface temperatures of devices used in cosmic space such as an artificial satellite, a probe, and a lunar rover widely range from a temperature below zero degrees to a high temperature, because effects of thermal irradiation from the sun and thermal release are great, unlike the earth surface surrounded by the atmosphere.

Accordingly, as such devices are exposed to a tough environment different from the environment on the earth, in order to keep equipment mounted on the devices smoothly functioning for a long period, a lubricating oil composition having low evaporativity and a high viscosity index and being excellent in fuel consumption saving performance and wear resistance is required.

As a method for improving fuel consumption saving performance, a method for decreasing viscosity of a lubricating oil composition and decreasing viscosity resistance of the lubricating oil composition to reduce the friction coefficient thereof, thereby suppressing energy loss is known.

Since lubricating oil compositions for lubricating equipment mounted on space devices are also required to maintain wear resistance and lubricity for a long period (low evaporativity), MAC oil (cyclopentane oil such as tris(2-octyldodecyl)cyclopentane) with a low vapor pressure is used as base oil of the main component thereof.

Perfluoroalkyl ether (PFAE) superior in low evaporativity and low-temperature flowability to MAC oil is also used as a main component of a lubricating oil composition mounted on space devices.

As a lubricant composition using MAC oil, Patent Literature 1 discloses a semisolid lubricant composition containing tris(2-octyldodecyl)cyclopentane, for example.

### Citation List

### Patent Literature

Patent Literature 1:JP H10-140169A

### Summary of Invention

### Technical Problem

However, the problem with MAC oil is that the viscosity index is low and the change in viscosity in response to temperature is large. In addition, it could not have been said that MAC oil is sufficient from viewpoints of lowering viscosity and of long life properties. The problem with PFAE is that reduction in friction coefficient and wear resistance are not sufficient.

The present invention has been made in view of the above-described problems, and an object thereof is to provide a compound, lubricant base oil, and a lubricating oil composition which have low viscosity and a high viscosity index and are excellent in wear resistance and reduction in friction coefficient.

### Solution to Problem

As a result of intensive studies, the present inventors have found that a certain compound can solve the above problems and completed the present invention.

That is, the present invention provides the following item [1].
[1] A compound represented by General Formula (A-1): 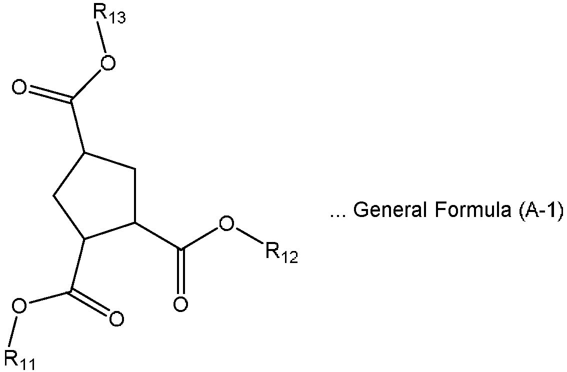 wherein R₁₁, R₁₂, and R₁₃ are each independently a chained alkyl group having 4 to 30 carbon atoms.

### Advantageous Effects of Invention

According to the present invention, a compound, lubricant base oil, and a lubricating oil composition which have low viscosity and a high viscosity index and are excellent in wear resistance and reduction in friction coefficient can be provided.

### Description of Embodiments

In the present specification, lower limit values and upper limit values described for a preferable numerical range (for example, a range of a content) in a step-by-step manner can be each independently combined. For example, based on a description of lower limit values "preferably 10 or more, more preferably 30 or more, and still more preferably 40 or more" and a description of upper limit values "preferably 90 or less, more preferably 80 or less, and still more preferably 70 or less", ranges such as "10 or more and 70 or less", "30 or more and 70 or less", and "40 or more and 80 or less" obtained by combining a lower limit value and an upper limit value each independently selected can be selected as preferable ranges. Based on the same descriptions, it is also possible to simply select a range such as "40 or more" or "70 or less" in which either a lower limit value or an upper limit value is defined, for example. The same applies to preferable ranges selectable based on a description like "preferably 10 or more and 90 or less, more preferably 30 or more and 80 or less, and still more preferably 40 or more and 70 or less" or "preferably 10 to 90, more preferably 30 to 80, and still more preferably 40 to 70". In the present specification, a description of a numerical range "10 to 90" has the same meaning as "10 or more and 90 or less", for example. Numerical values in descriptions of numerical ranges with "or more", "or less", "less than", and "more than" can also be arbitrarily combined.

In the present specification, the term "(meth)acrylate" is used to refer to both "acrylate" and "methacrylate", for example, and the same applies to other similar terms and similar representations.

A compound of the present embodiment is a compound represented by General Formula (A-1) below.

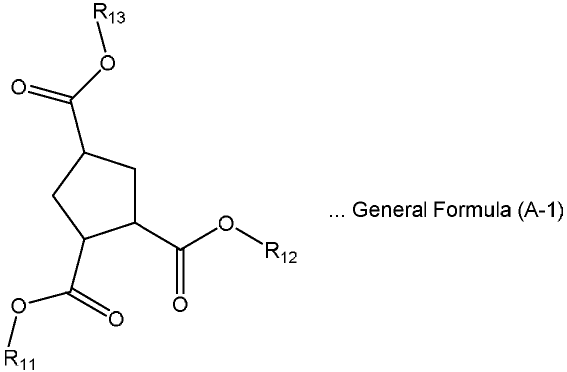

[In General Formula (A-1) above, R₁₁, R₁₂, and R₁₃ are each independently a chained alkyl group having 4 to 30 carbon atoms.]

The present inventors have made intensive studies to solve the above problems; as a result, the present inventors have found that a compound which has low viscosity and a high viscosity index and is excellent in wear resistance and reduction in friction coefficient can be obtained by providing a compound represented by General Formula (A-1) above.

Based on these findings, the present inventors have completed the present invention after a great deal of intensive studies.

### Compound

A compound of the present embodiment is a compound represented by General Formula (A-1) below.

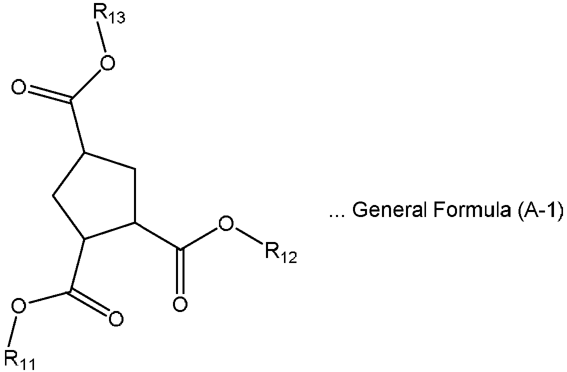

[In General Formula (A-1) above, R₁₁, R₁₂, and R₁₃ are each independently a chained alkyl group having 4 to 30 carbon atoms.]

In General Formula (A-1) above, R₁₁, R₁₂, and R₁₃ are each independently a chained alkyl group having 4 to 30 carbon atoms.

When the number of carbon atoms in the chained alkyl group is less than 4, the molecular weight of the compound is small, and low evaporativity becomes poor.

When the number of carbon atoms in the alkyl group exceeds 30, the molecular weight of the compound is increased; thus, viscosity is increased, and low-temperature flowability becomes poor.

The number of carbon atoms in R₁₁, R₁₂, and R₁₃ is preferably 8 to 28, more preferably 12 to 26, and still more preferably 16 to 24.

Examples of the chained alkyl group having 4 to 30 carbon atoms which may be selected as R₁₁, R₁₂, and R₁₃ include a butyl group, a pentyl group, a hexyl group, a heptyl group, an octyl group, a nonyl group, a decyl group, an undecyl group, a dodecyl group, a tridecyl group, a tetradecyl group, a pentadecyl group, a hexadecyl group, a heptadecyl group, an octadecyl group, an isostearyl group, a nonadecyl group, an icosyl group, a heneicosyl group, a docosyl group, a tricosyl group, a tetracosyl group, a pentacosyl group, a hexacosyl group, a heptacosyl group, an octacosyl group, a nonacosyl group, and a triacontyl group, and these groups may be a linear chain or a branched chain.

From the viewpoint of flowability, especially low-temperature flowability, it is preferable that at least one chained alkyl group of R₁₁, R₁₂, and R₁₃ have a branched chain, and it is more preferable that all chained alkyl groups have a branched chain.

When the chained alkyl group has a branched chain, the branching position is preferably the β-position from the viewpoint of synthesis.

When the chained alkyl group has a branched chain, the alkyl group preferably includes a 2-hexyldecyl group, a 2-octyldodecyl group, and a 2-decyltetradecyl group.

### Molecular weight

The molecular weight of the compound represented by General Formula (A-1) above is preferably 850 or more and 1250 or less, more preferably 860 or more and 1240 or less, and still more preferably 870 or more and 1230 or less from the viewpoints of maintaining lubricity and of low evaporativity.

### Density (15°C)

The density at 15°C of the compound represented by General Formula (A-1) above is preferably 0.8000 g/cm³ or more and 0.9500 g/cm³ or less, more preferably 0.8200 g/cm³ or more and 0.9400 g/cm³ or less, and still more preferably 0.8500 g/cm³ or more and 0.9300 g/cm³ or less from the viewpoints of maintaining lubricity and of low evaporativity.

The density at 15°C of the compound represented by General Formula (A-1) above can be measured in accordance with JIS K 2249-1:2011.

### Pour point

The pour point of the compound represented by General Formula (A-1) above is preferably -20°C or lower, more preferably -25°C or lower, and still more preferably -30°C or lower from the viewpoint of suppressing increase in viscosity resistance at low temperature.

The pour point of the compound represented by General Formula (A-1) above can be measured at 15°C in accordance with JIS K 2269:1987.

### 40°C kinematic viscosity, 100°C kinematic viscosity, and viscosity index

The 40°C kinematic viscosity of the compound represented by General Formula (A-1) above is preferably 2.0 mm²/s or more and 100.0 mm²/s or less, more preferably 20.0 mm²/s or more and 97.0 mm²/s or less, and still more preferably 50.0 mm²/s or more and 95.0 mm²/s or less from the viewpoints of low evaporativity and of suppressing power loss due to viscosity resistance.

The 100°C kinematic viscosity of the compound represented by General Formula (A-1) above is preferably 2.00 mm²/s or more and 15.00 mm²/s or less, more preferably 4.00 mm²/s or more and 15.00 mm²/s or less, and still more preferably 7.00 mm²/s or more and 14.50 mm²/s or less from the viewpoints of low evaporativity and of suppressing power loss due to viscosity resistance.

The viscosity index of the compound represented by General Formula (A-1) above is preferably 141 or more, more preferably 142 or more, and still more preferably 143 or more from the viewpoint of decreasing change in viscosity even when used in a space environment in which temperature ranges greatly vary like a low temperature environment and a high temperature environment.

The 40°C kinematic viscosity, the 100°C kinematic viscosity, and the viscosity index can be measured or calculated in accordance with JIS K 2283:2000.

### Friction coefficient

The friction coefficient of the compound represented by General Formula (A-1) above is preferably 0.065 or less, more preferably 0.064 or less, and still more preferably 0.063 or less when determined by the method described in the examples.

### Wear resistance

With respect to wear resistance of the compound represented by General Formula (A-1) above, the wear mark diameter is preferably 0.35 mm or less, more preferably 0.33 mm or less, and still more preferably 0.31 mm or less when determined by the method described in the examples.

### Method for synthesizing compound represented by General Formula (A-1)

The compound represented by General Formula (A-1) above can be synthesized by the following synthesis method, for example.

First, a cyclopentane derivative is synthesized by the method shown below.

A 2-substituted-5-norbomene compound represented by General Formula (1) is subjected to olefin oxidative cleavage reaction using an oxidant. The oxidant includes hydrogen peroxide, a periodic acid salt, a hypochlorous acid salt, tungsten oxide, osmium oxide, ruthenium oxide, and ruthenium chloride. Among these, a periodic acid salt and ruthenium chloride are preferable from the viewpoint of ease in handling. The amount of the periodic acid salt may be 1 molar equivalent or more based on a 5-norbomene-2-carboxylic acid ester but is more preferably 2 to 5 molar equivalents. The amount of ruthenium chloride may be a catalytic amount but is more preferably 0.001 to 0.1 molar equivalents based on a 5-norbomene-2-carboxylic acid ester. Reaction is carried out in a mixed solvent of ethyl acetate, acetonitrile, and water at 0°C to 30°C, for example. After completion of reaction, a dicarboxylic acid compound represented by General Formula (2) can be obtained at a yield of 50% to 90% through extraction with an organic solvent such as ethyl acetate and concentration.

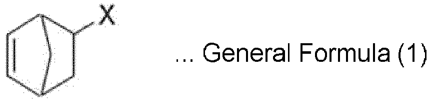

[In General Formula (1), X is an alkoxycarbonyl group.]

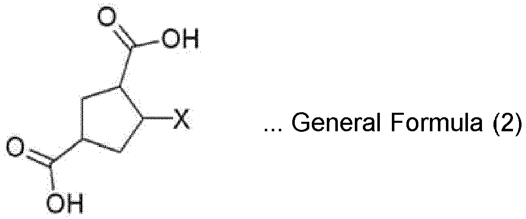

[In General Formula (2), X is an alkoxycarbonyl group.]

Thereafter, the compound represented by General Formula (A-1) above can be obtained by reacting the obtained cyclopentane derivative with a predetermined alcohol and esterifying same.

### Lubricant base oil

The compound represented by General Formula (A-1) above has low viscosity and a high viscosity index and is excellent in wear resistance and reduction in friction coefficient, and thus can be used as lubricant base oil for a lubricating oil composition described later.

The lubricant base oil includes the compound represented by General Formula (A-1) described above.

The content of the compound represented by General Formula (A-1) is preferably 50% by mass to 100% by mass, more preferably 70% by mass to 100% by mass, and still more preferably 80% by mass to 100% by mass, based on the total amount of the lubricant base oil.

### Lubricating oil composition

A lubricating oil composition of the present embodiment includes the compound represented by General Formula (A-1) described above as lubricant base oil.

The lubricating oil composition of the present embodiment includes at least the above-described lubricant base oil of the present invention but may contain other base oil together with the lubricant base oil within a range not impairing the effects of the present invention.

Examples of the other base oil include mineral oil and synthetic oil.

These may be used singly, or two or more kinds thereof may be used in combination.

Examples of the mineral oil include atmospheric residual oil obtained by distilling, at normal pressure, crude oil such as paraffin group crude oil, intermediate group crude oil, and naphthene group crude oil; distillate oil obtained by distilling the atmospheric residual oil at reduced pressure; and mineral oil obtained by subjecting the distillate oil to one or more refinement treatments such as solvent deasphalting, solvent extraction, hydrofinishing, hydrogenolysis, high hydrogenolysis, solvent dewaxing, catalytic dewaxing, and hydroisomerization dewaxing.

Examples of the synthetic oil include hydrocarbon-based oil, aromatic oil, ester-based oil, ether-based oil, and synthetic oil obtained by isomerizing wax (GTL wax) produced by a Fischer-Tropsch method. One kind thereof may be used alone, or two or more kinds thereof may be used in combination.

Examples of the hydrocarbon-based oil include poly-α-olefin (PAO) such as normal paraffin, isoparaffin, polybutene, polyisobutylene, a 1-decene oligomer, and a cooligomer of 1-decene and ethylene; and hydrogenated products thereof.

Examples of the aromatic oil include alkylbenzenes such as a monoalkylbenzene and a dialkylbenzene; and alkylnaphthalenes such as a monoalkylnaphthalene, a dialkylnaphthalene, and a polyalkylnaphthalene.

Examples of the ester-based oil include diester-based oil such as dibutyl sebacate, di-2-ethylhexyl sebacate, dioctyl adipate, diisodecyl adipate, ditridecyl adipate, ditridecyl glutarate, and methyl acetyl ricinoleate; aromatic ester-based oil such as trioctyl trimellitate, tridecyl trimellitate, and tetraoctyl pyromellitate; polyol ester-based oil such as trimethylol propane caprylate, trimethylol propane pelargonate, pentaerythritol-2-ethylhexanoate, and pentaerythritol pelargonate; and complex ester-based oil such as an oligoester of a polyhydric alcohol and a fatty acid mixture of a dibasic acid and a monobasic acid.

Examples of the ether-based oil include polyglycols such as polyethylene glycol, polypropylene glycol, a polyethylene glycol monoether, and a polypropylene glycol monoether; and phenyl ether-based oil such as a monoalkyl triphenyl ether, an alkyldiphenyl ether, a dialkyl diphenyl ether, pentaphenyl ether, tetraphenyl ether, a monoalkyl tetraphenyl ether, and a dialkyl tetraphenyl ether.

In the lubricating oil composition of the present embodiment, the content of the lubricant base oil including the compound represented by General Formula (A-1) is preferably 70% by mass or more, more preferably 80% by mass or more, still more preferably 90% by mass or more, and further preferably 95% by mass or more based on the total amount (100% by mass) of the lubricating oil composition.

In the lubricating oil composition of the present embodiment, the upper limit value of the content of the lubricant base oil including the compound represented by General Formula (A-1) may be 100% by mass based on the total amount (100% by mass) of the lubricating oil composition. Provided that when the lubricating oil composition includes a component (hereinafter, referred to as an "additional component") other than the lubricant base oil including the compound represented by General Formula (A-1), the upper limit value of the content of the lubricant base oil including the compound represented by General Formula (A-1) may be adjusted in relation to the additional component and is preferably 99% by mass or less and more preferably 98% by mass or less.

Hereinafter, each component included in the lubricating oil composition of the present embodiment will be described.

The content of the compound represented by General Formula (A-1) is preferably 60% by mass to 100% by mass, more preferably 70% by mass to 100% by mass, and still more preferably 80% by mass to 100% by mass based on the total amount of the lubricant base oil including the compound represented by General Formula (A-1).

In the lubricating oil composition of the present embodiment, the content of the lubricant base oil including the compound represented by General Formula (A-1) is not particularly limited but is preferably 60% by mass to 99.5% by mass, more preferably 70% by mass to 99.0% by mass, and still more preferably 80% by mass to 98.5% by mass based on the total amount (100% by mass) of the lubricating oil composition from the viewpoint of more easily exhibiting the effects of wear resistance and reduction in friction coefficient.

In the lubricating oil composition of the present embodiment, the content of the compound represented by General Formula (A-1) above is not particularly limited but is preferably 60% by mass to 99.5% by mass, more preferably 70% by mass to 99.0% by mass, and still more preferably 80% by mass to 98.5% by mass based on the total amount (100% by mass) of the lubricating oil composition from the viewpoint of more easily exhibiting the effects of wear resistance and reduction in friction coefficient.

Incidentally, the lubricant base oil including the compound represented by General Formula (A-1) may be used singly, or two or more kinds thereof may be used in combination. A preferable total content in the case of using two or more kinds is also the same as the above-described content.

### Additional component

The lubricating oil composition of the present embodiment may contain or may not contain an additional component other than the above-described components within a range not impairing the effects of the present invention, if needed.

Examples of the additional component include a viscosity index improver.

One kind of these components may be used singly, or two or more kinds thereof may be used in combination.

### Viscosity index improver

When the lubricating oil composition of the present embodiment contains a viscosity index improver, the viscosity index of the lubricating oil composition can be improved. Consequently, change in viscosity can be reduced even when used in a space environment in which temperature ranges greatly vary like a low temperature environment and a high temperature environment.

Examples of the viscosity index improver include polymers such as a non-dispersive poly(meth)acrylate and a dispersive poly(meth)acrylate.

One kind thereof may be used singly, or two or more kinds thereof may be used in combination.

The mass average molecular weight (Mw) of these viscosity index improvers is usually 5,000 to 1,000,000, preferably 6,000 to 100,000, and more preferably 10,000 to 50,000 but is appropriately set according to the type of the polymer.

In the present specification, the mass average molecular weight (Mw) of each component is a value in terms of standard polystyrene measured by a gel permeation chromatography (GPC) method.

The content of the additional component described above can be appropriately adjusted within a range not impairing the effects of the present invention, but is usually 0.001 % by mass to 15% by mass, preferably 0.005% by mass to 10% by mass, more preferably 0.01% by mass to 7% by mass, and still more preferably 0.03% by mass to 5% by mass based on the total amount (100% by mass) of the lubricating oil composition for each component.

In the present specification, an additive as the additional component may be blended with the other components in a form of a solution in which the additive is diluted with and dissolved in part of the above-described lubricant base oil, taking into consideration handleability. In such a case, the above-described content of the additive as the additional component means a content in terms of effective ingredient (in terms of resin) excluding the diluent, in the present specification.

### Physical property values of lubricating oil composition

### Density (15°C)

The density of the lubricating oil composition at 15°C is preferably 0.8000 g/cm³ or more and 0.9500 g/cm³ or less, more preferably 0.8200 g/cm³ or more and 0.9400 g/cm³ or less, and still more preferably 0.8500 g/cm³ or more and 0.9300 g/cm³ or less.

The density of the lubricating oil composition at 15°C can be measured in accordance with JIS K 2249-1:2011.

### Pour point

The pour point of the lubricating oil composition is preferably -20°C or lower, more preferably -25°C or lower, and still more preferably -30°C or lower from the viewpoint of suppressing increase in viscosity resistance at low temperature.

The pour point of the lubricating oil composition can be measured at 15°C in accordance with JIS K 2269:1987.

### 40°C kinematic viscosity, 100°C kinematic viscosity, and viscosity index

The 40°C kinematic viscosity of the lubricating oil composition is preferably 2.0 mm²/s or more and 100.0 mm²/s or less, more preferably 20.0 mm²/s or more and 97.0 mm²/s or less, and still more preferably 50.0 mm²/s or more and 95.0 mm²/s or less from the viewpoints of low evaporativity and of suppressing power loss due to viscosity resistance.

The 100°C kinematic viscosity of the lubricating oil composition is preferably 2.00 mm²/s or more and 15.00 mm²/s or less, more preferably 4.00 mm²/s or more and 15.00 mm²/s or less, and still more preferably 7.00 mm²/s or more and 14.50 mm²/s or less from the viewpoints of low evaporativity and of suppressing power loss due to viscosity resistance.

The viscosity index of the lubricating oil composition is preferably 141 or more, more preferably 142 or more, and still more preferably 143 or more from the viewpoint of decreasing change in viscosity even when used in a space environment in which temperature ranges greatly vary like a low temperature environment and a high temperature environment.

The 40°C kinematic viscosity, the 100°C kinematic viscosity, and the viscosity index can be measured or calculated in accordance with JIS K 2283:2000.

In addition, when the lubricating oil composition is a mixture of two or more kinds of base oil, the kinematic viscosity and the viscosity index of the mixture are preferably within the above-described ranges.

### Friction coefficient

The friction coefficient of the lubricating oil composition is preferably 0.065 or less, more preferably 0.064 or less, and still more preferably 0.063 or less when determined by the method described in the examples.

### Wear resistance

With respect to wear resistance of the lubricating oil composition, the wear mark diameter is preferably 0.35 mm or less, more preferably 0.33 mm or less, and still more preferably 0.31 mm or less when determined by the method described in the examples.

### Use of lubricating oil composition

Since the lubricating oil composition of the present embodiment has low viscosity and a high viscosity index and is excellent in wear resistance and reduction in friction coefficient, the lubricating oil composition of the present embodiment can be suitably used for equipment mounted on devices used in cosmic space such as an artificial satellite, a probe, and a lunar rover. In addition, since the lubricating oil composition of the present embodiment is successfully used in a high-vacuum environment, the lubricating oil composition of the present embodiment can be suitably used for devices for producing a semiconductor, a flat panel display such as a liquid crystal display and an organic EL display, and a solar cell panel.

Incidentally, the lubricating oil composition of the present embodiment is suitably used for application as a lubricating oil composition for devices used in cosmic space but may be used for other application.

According to the present embodiment, use of the above-described lubricating oil composition as space lubricating oil is provided.

### Lubricating oil composition production method

The present embodiment provides a lubricating oil composition production method including a step of mixing the compound represented by General Formula (A-1) below.

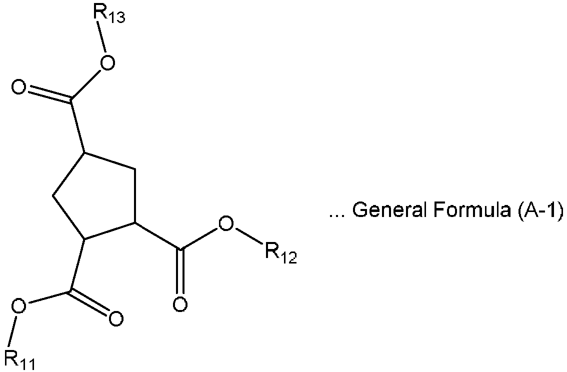

[In General Formula (A-1) above, R₁₁, R₁₂, and R₁₃ are each independently a chained alkyl group having 4 to 30 carbon atoms.]

The production method may further include a step of adding the other components described above.

### Grease composition

The lubricating oil composition of the present embodiment can also be used as a grease composition when the lubricating oil composition of the present embodiment includes the lubricant base oil described above and a thickener.

Since cosmic space is a micro gravity environment, lubricating oil gravity feeding methods generally used on the earth cannot be applied. However, lubricity can be maintained without feeding lubricating oil during use by applying the lubricating oil composition of the present embodiment as a grease composition.

The grease composition of the present embodiment contains at least the above-described lubricant base oil of the present invention but may contain other base oil together with the lubricant base oil within a range not impairing the effects of the present invention

The other base oil that may be contained in the grease composition of the present embodiment is the same as the lubricant base oil that may be contained in the above-described lubricating oil composition of the present invention.

The grease composition of the present embodiment may contain an additional component other than the above-described components, if needed.

The additional component that may be contained in the grease composition of the present embodiment is the same as the additional component that may be contained in the above-described lubricating oil composition of the present invention.

According to an aspect of the present invention, items [1] to [7] below are provided.
[1] A compound represented by General Formula (A-1): 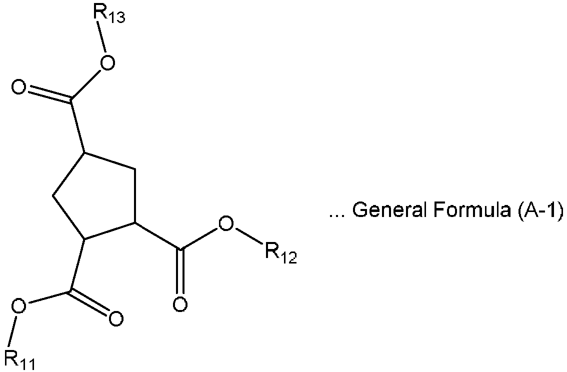 wherein R₁₁, R₁₂, and R₁₃ are each independently a chained alkyl group having 4 to 30 carbon atoms.
[2] The compound according to [1] above, in which at least one chained alkyl group of R₁₁, R₁₂, and R₁₃ in General Formula (A-1) above has a branched chain.
[3] The compound according to [1] or [2] above, having a molecular weight of 850 or more and 1250 or less.
[4] The compound according to any one of [1] to [3] above, in which kinematic viscosity at 100°C is 14.50 mm²/s or less.
[5] The compound according to any one of [1] to [4] above, having a viscosity index of 141 or more.
[6] Lubricant base oil including the compound according to any one of [1] to [5] above.
[7] A lubricating oil composition including the lubricant base oil according to [6] above.

### Examples

The present invention will be more specifically described by the following examples, but the present invention is not limited to the following examples.

### Synthesis of compound

Compounds of Examples 1 to 6 and Comparative Examples 1 to 5 are synthesized by the method described later.

### Example 1

### Production of cyclopentane-1,2,4-tricarboxylic acid-1,2,4-tri-2-octyldodecane ester

In a 500-mL glass reaction vessel, were placed 28.8 g of sodium periodate, 99 mL of water, 66 mL of ethyl acetate, and 66 mL of acetonitrile, followed by stirring at an internal temperature of 18°C to 19°C in a water bath. After adding 25 mg of ruthenium chloride n-hydrate thereto, 5.0 g of methyl 5-norbomene-2-carboxylate was added dropwise. After stirring the mixture for 4.5 hours, 200 mL of water was added, followed by liquid separation. The water layer was extracted with ethyl acetate, then mixed with the organic layer, and washed with 50 mL of 2M hydrochloric acid. After further washing with 100 mL of a 0.5M sodium thiosulfate aqueous solution and 50 mL of saturated saline, the obtained organic layer was dried with magnesium sulfate anhydride. After filtration, 6.5 g of 1-methyl cyclopentane-1,2,4-tricarboxylate was obtained as a brown oily product through concentration.

Thereafter, this crude product, 29.6 g of 2-octyl dodecanol, 0.4 g of p-toluene sulfonic acid, and 4 mL of toluene were placed in a 200-mL glass reaction vessel, followed by stirring at 180°C for 14 hours. After cooling to room temperature, the product was washed with 100 mL of a 1M sodium hydroxide aqueous solution, followed by extraction with ethyl acetate and drying with magnesium sulfate anhydride. After filtration, a target product was obtained through concentration and purification using silica gel column chromatography.

The obtained compound of Example 1 was 27.1 g and 0.026 mol. The structure of the compound of Example 1 is shown in Structural Formula (A-1).

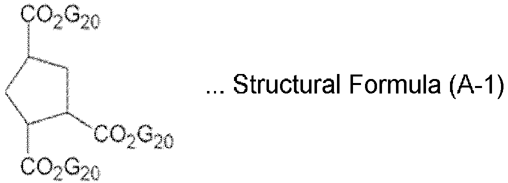

[In Structural Formula (A-1) above, G₂₀ is a 2-octyldodecyl group.]

### Example 2

The compound of Example 2 was obtained in the same manner as in Example 1 except that 2-octyldodecanol was changed to 2-hexyldecanol in Example 1.

The obtained compound of Example 2 was 23.6 g and 0.027 mol. The structure of the compound of Example 2 is shown in Structural Formula (A-2).

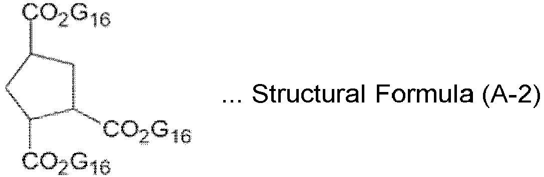

[In Structural Formula (A-2) above, G₁₆ is a 2-hexyldecyl group.]

### Example 3

The compound of Example 3 was obtained in the same manner as in Example 1 except that 2-octyldodecanol was changed to 2-decyltetradecanol in Example 1.

The obtained compound of Example 3 was 29.1 g and 0.024 mol. The structure of the compound of Example 3 is shown in Structural Formula (A-3).

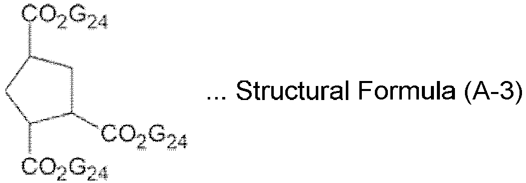

[In Structural Formula (A-3) above, G₂₄ is a 2-decyltetradecyl group.]

### Example 4

The compound of Example 4 was obtained in the same manner as in Example 1 except that 2-octyldodecanol was changed to a mixture of 2-decyltetradecanol and 2-hexyldecanol (at a molar ratio of 2: 1) in Example 1.

The obtained compound of Example 4 was 27.5 g and 0.025 mol. The structure of the compound of Example 4 is shown in Structural Formula (A-4).

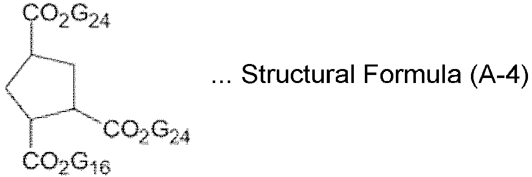

[In Structural Formula (A-4) above, G₁₆ is a 2-hexyldecyl group, and G₂₄ is a 2-decyltetradecyl group.]

### Example 5

The compound of Example 5 was obtained in the same manner as in Example 1 except that 2-octyldodecanol was changed to a mixture of 2-octyldodecanol, 2-decyltetradecanol, and 2-hexyldecanol (at a molar ratio of 1:1:1) in Example 1.

The obtained compound of Example 5 was 26.1 g and 0.025 mol. The structure of the compound of Example 5 is shown in Structural Formula (A-5).

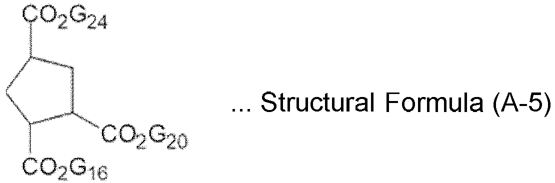

[In Structural Formula (A-5) above, G₁₆ is a 2-hexyldecyl group, G₂₀ is a 2-octyldodecyl group, and G₂₄ is a 2-decyltetradecyl group.]

### Example 6

The compound of Example 6 was obtained in the same manner as in Example 1 except that 2-octyldodecanol was changed to a mixture of 2-octyldodecanol and 2-decyltetradecanol (at a molar ratio of 2:1) in Example 1.

The obtained compound of Example 6 was 25.3 g and 0.023 mol. The structure of the compound of Example 6 is shown in Structural Formula (A-6).

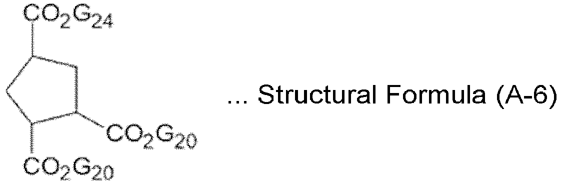

[In Structural Formula (A-6) above, G₂₀ is a 2-octyldodecyl group, and G₂₄ is a 2-decyltetradecyl group.]

### Comparative Example 1

MAC oil (= 1,2,4-(tris(2-octyldodecyl)cyclopentane) (manufactured by Nye Lubricants, Inc., product name: 2001A)) was used as the compound of Comparative Example 1. The structure of the compound of Comparative Example 1 is shown in Structural Formula (A'-1).

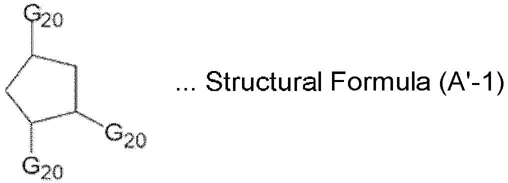

[In Structural Formula (A'-1) above, G₂₀ is a 2-octyldodecyl group.]

### Comparative Example 2

The compound of Comparative Example 2 was obtained in the same manner as in Example 1 except that 1-methyl cyclopentane-1,2,4-tricarboxylate was changed to cyclohexane-1,2,4-tricarboxylic acid in Example 1.

The obtained compound of Comparative Example 2 was 24.3 g and 0.023 mol. The structure of the compound of Comparative Example 2 is shown in Structural Formula (A'-2).

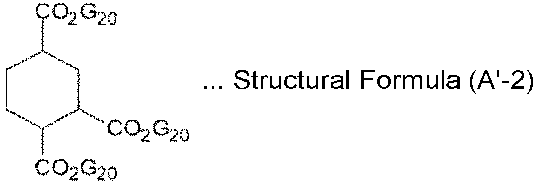

[In Structural Formula (A'-2) above, G₂₀ is a 2-octyldodecyl group.]

### Comparative Example 3

The compound of Comparative Example 3 was obtained in the same manner as in Example 1 except that 1-methyl cyclopentane-1,2,4-tricarboxylate was changed to trimellitic anhydride in Example 1.

The obtained compound of Comparative Example 3 was 27.4 g and 0.026 mol. The structure of the compound of Comparative Example 3 is shown in Structural Formula (A'-3).

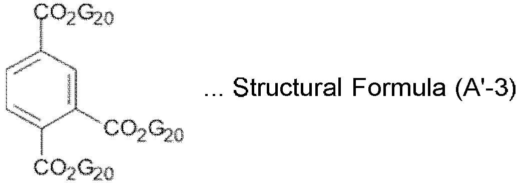

[In Structural Formula (A'-3) above, G₂₀ is a 2-octyldodecyl group.]

### Comparative Example 4

The compound of Comparative Example 4 was obtained in the same manner as in Example 1 except that 1-methyl cyclopentane-1,2,4-tricarboxylate was changed to cyclopentane-1,2,3,4-tetracarboxylic acid in Example 1.

The obtained compound of Comparative Example 4 was 28.7 g and 0.021 mol. The structure of the compound of Comparative Example 4 is shown in Structural Formula (A'-4).

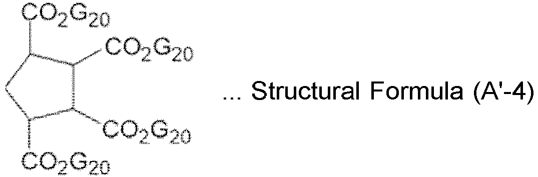

[In Structural Formula (A'-4) above, G₂₀ is a 2-octyldodecyl group.]

### Comparative Example 5

The compound of Comparative Example 5 was obtained in the same manner as in Example 2 except that 1-methyl cyclopentane-1,2,4-tricarboxylate was changed to cyclopentane-1,2,3,4-tetracarboxylic acid in Example 2.

The obtained compound of Comparative Example 5 was 26.0 g and 0.023 mol. The structure of the compound of Comparative Example 5 is shown in Structural Formula (A'-5).

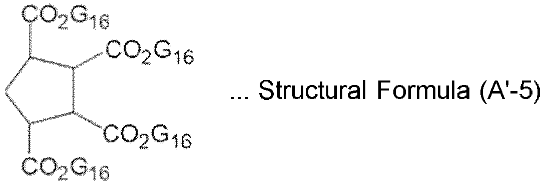

[In Structural Formula (A'-5) above, G₁₆ is a 2-hexyldecyl group.]

The following measurement and evaluation were conducted on each compound obtained. Results are show in Tables 1 and 2.

### Pour point

The pour point of each compound was measured in accordance with JIS K 2269:1987.

### Density (15°C)

The density at 15°C of each compound was measured in accordance with JIS K 2249-1:2011.

### Evaluation of 40°C kinematic viscosity, 100°C kinematic viscosity, and viscosity index

The 40°C kinematic viscosity, the 100°C kinematic viscosity, and the viscosity index of each compound were measured or calculated in accordance with JIS K 2283:2000.

Note that 40°C kinematic viscosity of 100.0 mm²/s or less, 100°C kinematic viscosity of 15.0 mm²/s or less, and a viscosity index of 141 or more were rated as acceptable.

### Evaluation of friction coefficient reduction effect and wear resistance

The friction coefficient and the wear mark diameter in the case of using a lubricating oil composition prepared were measured using reciprocating friction-testing machine TE77 (manufactured by Phoenix Tribology Ltd.) under the following conditions.
Test piece: ball upper part (SUJ2), disc lower part (SUJ2)
Amplitude: 10.0 mm
Frequency: 10 Hz
Load: 50 N
Temperature: 100°C
Test time: 20 minutes

**Table 1**

| | | | Examples | | |
|---|---|---|---|---|---|
| | | | 1 | 2 | 3 |
| Compound | Molecular formula | - | C₆₈H₁₃₀O₆ | C₅₆H₁₀₆O₆ | C₈₀H₁₅₄O₆ |
| Physical property values | Molecular weight | - | 1044 | 875 | 1212 |
| | Density (15°C) | g/cm³ | 0.9042 | 0.9167 | 0.8972 |
| | Pour point | °C | < -40 | < -40 | < -40 |
| Evaluation results | 40°C kinematic viscosity | mm²/s | 72.30 | 57.30 | 91.05 |
| | 100°C kinematic viscosity | mm2/s | 11.60 | 9.32 | 14.10 |
| | Viscosity index | - | 155 | 145 | 159 |
| | Friction coefficient | - | 0.053 | 0.060 | 0.049 |
| | Wear mark diameter | mm | 0.27 | 0.28 | 0.28 |

**Table 1 (continued)**

| | | | Examples | | |
|---|---|---|---|---|---|
| | | | 4 | 5 | 6 |
| Compound | Molecular formula | - | C₇₂H₁₃₈O₆ | C₆₈H₁₃₀O₆ | C₇₂H₁₃₈O₆ |
| Physical property values | Molecular weight | - | 1100 | 1044 | 1100 |
| | Density (15°C) | g/cm³ | 0.8999 | 0.9055 | 0.9019 |
| | Pour point | °C | < -40 | < -40 | < -40 |
| Evaluation results | 40°C kinematic viscosity | mm2/s | 83.32 | 73.20 | 80.20 |
| | 100°C kinematic viscosity | mm2/s | 13.10 | 11.69 | 12.65 |
| | Viscosity index | - | 158 | 154 | 157 |
| | Friction coefficient | - | 0.048 | 0.055 | 0.060 |
| | Wear mark diameter | mm | 0.24 | 0.26 | 0.26 |

**Table 2**

| | | | Comparative Examples | | |
|---|---|---|---|---|---|
| | | | 1 | 2 | 3 |
| Compound | Molecular formula | - | C₆₅H₁₃₀ | C₆₉H₁₃₂O₆ | C₆₉H₁₂₆O₆ |
| Physical property values | Molecular weight | - | 912 | 1058 | 1052 |
| | Density (15°C) | g/cm³ | 0.8459 | 0.9087 | 0.9163 |
| | Pour point | °C | < -40 | < -40 | < -40 |
| Evaluation results | 40°C kinematic viscosity | mm²/s | 103.7 | 114.1 | 142.3 |
| | 100°C kinematic viscosity | mm2/s | 13.96 | 15.07 | 17.35 |
| | Viscosity index | - | 136 | 136 | 134 |
| | Friction coefficient | - | 0.075 | 0.057 | 0.058 |
| | Wear mark diameter | mm | 0.38 | 0.23 | 0.25 |

**Table 2 (continued)**

| | | | Comparative Examples | |
|---|---|---|---|---|
| | | | 4 | 5 |
| Compound | Molecular formula | - | C₈₉H₁₇₀O₈ | C₇₃H₁₃₈O₈ |
| Physical property values | Molecular weight | - | 1368 | 1144 |
| | Density (15°C) | g/cm³ | 0.9068 | 0.9187 |
| | Pour point | °C | < -40 | < -40 |
| Evaluation results | 40°C kinematic viscosity | mm2/s | 108.9 | 94.55 |
| | 100°C kinematic viscosity | mm2/s | 15.37 | 13.12 |
| | Viscosity index | - | 149 | 139 |
| | Friction coefficient | - | 0.056 | 0.061 |
| | Wear mark diameter | mm | 0.23 | 0.23 |

As can be seen from Table 1, the compounds of Examples 1 to 6 had 40°C kinematic viscosity and 100°C kinematic viscosity as low as 100.0 mm²/s or less and 15.0 mm²/s or less, respectively, and had a viscosity index as high as 141 or more. In addition, the friction coefficients were 0.065 or less, the wear mark diameters were 0.35 mm or less, and excellent wear resistance and excellent reduction in friction coefficient were provided.

Accordingly, it has been found that the compounds of Examples 1 to 6 are useful as lubricant base oil.

## Claims

1. A compound represented by General Formula (A-1): 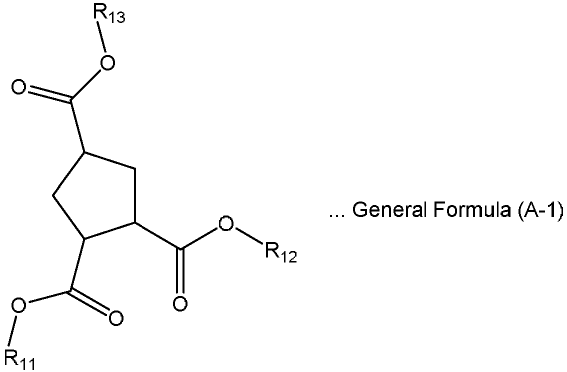 wherein R₁₁, R₁₂, and R₁₃ are each independently a chained alkyl group having 4 to 30 carbon atoms.

2. The compound according to claim 1, wherein at least one chained alkyl group of R₁₁, R₁₂, and R₁₃ in General Formula (A-1) has a branched chain.

3. The compound according to claim 1 or 2, having a molecular weight of 850 or more and 1250 or less.

4. The compound according to any one of claims 1 to 3, wherein kinematic viscosity at 100°C is 14.50 mm²/s or less.

5. The compound according to any one of claims 1 to 4, having a viscosity index of 141 or more.

6. Lubricant base oil comprising the compound according to any one of claims 1 to 5.

7. A lubricating oil composition comprising the lubricant base oil according to claim 6.
